# FASCICULE DE BREVET EUROPEEN

(11) **EP 2 793 818 B1**
(45) Date de publication et mention de la délivrance du brevet: **12.07.2017**
(21) Numéro de dépôt: 12816289.8
(22) Date de dépôt: 21.12.2012
(51) Int. Cl.: A61K 8/34, A61K 8/37, A61K 8/55, A61Q 19/00, A61Q 19/08

(54) **COMPOSITION HYDRATANTE**
FEUCHTIGKEITSSPENDENDE ZUSAMMENSETZUNG
MOISTURIZING COMPOSITION

(30) Priorité: 23.12.2011 FR 1162423; 30.01.2012 US 201261592370 P
(43) Date de publication de la demande: 29.10.2014
(73) Titulaire: L'Oréal, 75008 Paris (FR)
(72) Inventeur: LEREBOUR, Géraldine, 78350 Les Loges (FR); MARION, Catherine, 92160 Antony (FR)
(74) Mandataire: Lavoix
(86) Numéro de dépôt international: PCT/EP2012/076787
(87) Numéro de publication internationale: WO 2013/093069

(56) Documents cités:
- EP-A1- 2 243 462
- WO-A2-2007/103555
- DE-A1-102006 046 076
- FR-A1- 2 795 956

## Description

La présente invention concerne le domaine de la cosmétique, et en particulier de l'hydratation de la peau. La présente invention concerne en particulier une méthode de soin cosmétique permettant d'atténuer les stries de déshydratation, notamment au niveau du visage ou des yeux.

Les stries de déshydratation peuvent apparaître au niveau cutané, dans des cas de sécheresse cutanée, d'autant plus avec le vieillissement cutané. Ces stries peuvent être considérées comme disgracieuses en particulier sur les zones les plus visibles du corps humain comme les mains ou le visage, notamment au niveau péri-oculaire.

Le contour de l'oeil est une zone particulièrement fragile et complexe de par sa structure. Cette zone est beaucoup plus mince qu'au niveau du reste du visage. C'est une zone très sollicitée puisque la paupière est en mouvement afin d'assurer une hydratation permanente de la cornée. Le tissu cutané du contour de l'oeil est très élastique et plus lâche que la plupart des autres parties du corps et du visage, ce qui rend cette zone particulièrement fragile et sujette à l'apparition de ridules ou stries de déshydratation.

Afin de lutter contre l'apparition naturelle de ridules ou stries de déshydratation, il est connu d'appliquer sur la peau des compositions cosmétiques favorisant l'hydratation des zones d'application. L'amélioration de l'hydratation de la peau est donc une composante d'un traitement anti-rides.

De nombreuses compositions cosmétiques sont connues pour améliorer l'hydratation de la peau. Cependant il existe un besoin de nouvelles compositions présentant une bonne efficacité hydratante tout en ayant de bonnes propriétés sensorielles, comme un toucher soyeux. Des compositions cosmétiques hydratantes peuvent malheureusement en outre faire apparaître un effet dit de « tôle ondulée » inesthétique, malgré l'effet hydratant, se caractérisant par des plis à la surface cutanée, sorte d'effet fripé, la peau se rétractant en créant de fausses rides.

Ainsi la présente invention a pour but de fournir une composition, notamment cosmétique, notamment hydratante, en particulier pour atténuer les stries de déshydratation de la peau, et qui présenteraient de préférence un effet « tôle ondulée » inexistant ou amoindris.

La présente invention a aussi pour but de fournir une composition, notamment cosmétique présentant un toucher satisfaisant, et de préférence agréable, soyeux, notamment lors de son application sur la peau. En particulier la présente invention vise à fournir une composition, notamment cosmétique possédant une texture, un comportement à l'étalement, une vitesse de pénétration et un toucher de peau convenables.

Il a été découvert de manière surprenante qu'une composition, notamment cosmétique, comprenant au moins trois composés chimiques particuliers permettait de satisfaire aux buts énoncés ci-dessus.

L'invention concerne plus précisément une composition, notamment cosmétique, comprenant, dans un milieu physiologiquement acceptable, au moins du caprylate de glycéryl, du 1-3-propanediol et de l'acide phytique ou l'un de ses sels.

Les inventeurs ont découvert qu'une composition, notamment cosmétique, comprenant cette association ou combinaison de caprylate de glycérol (dénommé aussi caprylate de glycéryl), de 1,3-propanediol et d'acide phytique ou l'un de ses sels permet d'atténuer les stries de déshydratation. Cette composition, lorsqu'elle est appliquée sur la peau, conduit à un effet « tôle ondulée » inexistant ou amoindris par rapport à une composition ne comprenant pas cette association ou combinaison.

Par ailleurs, la composition selon l'invention présente un toucher agréable, notamment lors, de son application sur la peau. La composition de l'invention possède une texture, un comportement à l'étalement, une vitesse de pénétration et un toucher de peau satisfaisants. En particulier la composition de l'invention laisse subsister un très léger film gras et soyeux, ce qui est souhaité.

Le caprylate de glycéryl est un monoester de glycérine et acide caprylique. A titre d'exemple, peut être cité le produit vendu sous la référence commerciale dermosoft^{®} GMCY par la société DR STRAETMANS GmbH.

Le 1-3-propanediol peut être obtenu avantageusement auprès de la société DUPONT TATE AND LYLE BIO PRODUCTS sous la référence commerciale Zemea® Propanediol.

De préférence, le sel d'acide phytique est un sel alcalin d'acide phytique, et encore de préférence est le phytate de sodium. Le phytate de sodium peut avantageusement être obtenu auprès de la société DR STRAETMANS GmbH sous la référence commerciale dermofeel® PA-3.

Avantageusement la composition de l'invention comprend au plus 1,5 % en poids de caprylate de glycéryl, par rapport au poids total de la composition.

Avantageusement la composition de l'invention comprend au plus 25 % en poids de 1,3-propanediol, par rapport au poids total de la composition.

Avantageusement la composition de l'invention comprend au plus 0,3 % en poids d'acide phytique ou l'un de ses sels, par rapport au poids total de la composition.

Avantageusement, la composition de l'invention comprend :
- au plus ,5 % en poids de caprylate de glycéryl, par rapport au poids total de la composition ; et
- au plus 25 % en poids de 1,3-propanediol, par rapport au poids total de la composition ; et
- au plus 0,3 % en poids d'acide phytique ou l'un de ses sels, et en particulier de phytate de sodium, par rapport au poids total de la composition.

En particulier la présente invention comprend au plus 1,5 %, de préférence de 0,01% à 5 % en poids, et encore de préférence de 0,1% à 1,5 % en poids de caprylate de glycéryl par rapport au poids total de la composition.

En particulier la présente invention comprend au plus 25 %, de préférence de 0,1% à 25 % en poids, et encore de préférence de 0,5 à 10 % en poids de 1,3-propanediol par rapport au poids total de la composition.

En particulier la présente invention comprend au plus 0,3 % , de préférence de 0,01% à 1,5% en poids, et encore de préférence de 0,05 à 0,3 %, en poids d'acide phytique ou l'un de ses sels, et en particulier de phytate de sodium, par rapport au poids total de la composition.

Selon une variante particulière, la composition de l'invention comprend en outre un alcool, en particulier en C₂-C₈, autre que le 1,3-propanediol, et de préférence comprend de l'alcool éthylique. De préférence la composition comprend de 0,1 à 25%, et de préférence de 0,1 à 10%, et encore de préférence de 0,1 % à 5 %, en poids d'alcool autre que le 1,3-propanediol, et de préférence d'alcool éthylique, par rapport au poids total de la composition.

Selon une variante, la composition de l'invention est exempte de perlite.

La perlite est un verre naturel d'origine volcanique, de couleur gris-clair ou noir brillant, résultant du refroidissement rapide de la lave et qui se présente sous forme de petites particules ressemblant à de la perle. Les particules de perlite sont notamment disponibles dans le commerce auprès de la société WORLD MINERALS EUROPE sous la dénomination commerciale Perlite P1430, Perlite P2550 ou Perlite P2040. La perlite est exclue selon cette variante car elle peut être utilisée dans des compositions contraires au but de l'invention et à l'objectif d'hydratation de la peau.

La composition selon la présente invention est de préférence une composition cosmétique ou dermatologique, et tout préférentiellement une composition cosmétique.

Par « milieu physiologiquement acceptable », on entend désigner un milieu compatible avec les matières et/ou les fibres kératiniques d'êtres humains, comme par exemple, de manière non limitative, la peau, les muqueuses, les ongles, le cuir chevelu et/ou les cheveux.

Ce milieu physiologiquement acceptable comprend généralement de l'eau, éventuellement en mélange ou non avec un ou plusieurs solvants organiques.

La composition selon l'invention peut également comprendre une phase grasse, qui peut comprendre des huiles, des gommes, des cires usuellement utilisées dans le domaine d'application considéré.

De préférence la composition comprend en outre un ou plusieurs corps gras, comme l'alcool cétylstéarylique, le dicaprylyl cabronate, de l'huile de tournesol, du beurre de karité, un mélange d'origine végétale de lécithine, d'acides gras et d'alcool gras, un ou plusieurs polymères comme une gomme de scléroglucane, du xanthane, un ou plusieurs polysaccharides, comme le glucose, le mannosse et/ou l'acide glucuronique, un ou plusieurs solvants comme le glycérol, l'eau, un ou plusieurs tensioactifs comme un ester citrique de stéarate de glycérol, et/ou plusieurs vitamines comme le tocophérol (alpha, beta, gamma et/ou delta), et/ou un ou plusieurs conservateurs, comme l'acide benzylique. De préférence la composition de l'invention comprend de 5 à 35 % en poids de corps gras, de 0 à 5% en poids de polymère(s), de 65 à 95% de solvant(s), et de 0 à 5% de tensioactif(s). Le terme « 0% » couvre les variantes où le composé est absent, et où il est présent en traces détectables.

Les compositions cosmétiques selon l'invention peuvent contenir les additifs habituels en cosmétique : pigments, colorants, actifs biologiques (anti-âge, anti-peaux grasse, éclaircissant, blanchissant, anti-oxydants, etc ...), filtres solaires, polymères filmogènes, huiles, corps gras, hydratants, émollients, solvants, tensioactifs, vitamines, et/ou conservateurs, ou d'autres excipients cosmétiques.

Les compositions selon l'invention peuvent se présenter sous toute forme galénique qui convienne à l'usage selon la présente invention, et en particulier sous la forme de gels, de sérums, d'émulsions directes, inverses ou multiples, de sticks, de coulés à chaud, de poudres libres ou compactées, ou de crèmes.

Avantageusement, la composition selon l'invention se présente sous la forme d'une émulsion, en particulier sous forme d'une émulsion Huile-dans-Eau (H/E).

Selon une variante, la composition, qui est de préférence exempte de perlite, n'est pas un déodorant.

L'invention concerne également une méthode de préparation d'une composition cosmétique selon l'invention, la méthode comprenant le mélange de caprylate de glycéryl, de 1,3-propanediol et de l'acide phytique ou l'un de ses sels, et en particulier du phytate de sodium, avec des excipients cosmétiques, et éventuellement avec un ou plusieurs principes actifs cosmétiques.

L'invention concerne aussi l'utilisation d'une composition, notamment cosmétique, selon l'invention pour hydrater la peau. En particulier la composition de l'invention est appliquée sur au moins une zone de la peau présentant des stries de déshydratation, comme les mains ou le visage, en particulier le front, les joues, ou le contour d'un oeil (péri-oculaire), et en particulier la patte d'oie, la zone en dessous de l'oeil (poche), les paupières, le cou, les pieds, les jambes, les bras, les avant-bras, ou une autre zone sèche du corps.

En particulier l'invention concerne l'utilisation d'une composition, notamment cosmétique, comprenant, dans un milieu physiologiquement acceptable, au moins du caprylate de glycéryl, du 1,3-propanediol et de l'acide phytique ou l'un de ses sels, et en particulier le phytate de sodium, pour hydrater la peau.

L'invention concerne encore une méthode de soin cosmétique comprenant l'application sur la peau d'une composition, notamment cosmétique, selon l'invention, comprenant dans un milieu physiologiquement acceptable, au moins du caprylate de glycéryl, du 1,3-propanediol et de l'acide phytique ou l'un de ses sels, et en particulier du phytate de sodium. Typiquement, la méthode de l'invention comprend l'application d'une composition de l'invention sur une zone cutanée en ayant besoin.

L'ensemble des variantes, modes de réalisation, préférences, ou aspects particuliers des compositions de l'invention s'appliquent aux utilisations et méthodes telles que définies dans l'invention.

Selon une variante particulière, la composition est exempte de perlite.

Avantageusement, la méthode réduit la visibilité des rides et ridules de la peau, notamment sur le visage, et en particulier autour des yeux et sur les paupières.

Avantageusement, la composition de l'invention est appliquée sur une peau sèche et/ou âgée Selon une variante préférée, ces méthodes comprennent l'application de la composition sur une zone choisie parmi les mains, le visage, en particulier le front, les joues, ou le contour d'un oeil (péri-oculaire), et en particulier la patte d'oie, la zone en dessous de l'oeil (poche), les paupières , le cou, les pieds, les jambes, les bras, les avant-bras, ou une autre zone sèche du corps.

De préférence, ladite zone cutanée présente de stries de déshydratation.

De préférence la composition de l'invention est appliquée sur une zone autre que les aisselles.

La présente invention concerne en particulier une méthode de soin des peaux sèches.

Des exemples concrets, mais nullement limitatifs, illustrant l'invention, vont maintenant être donnés.

Dans les exemples, tous les pourcentages sont donnés en poids, sauf indication contraire, et la température est exprimée en degré Celsius sauf indication contraire, et la pression est la pression atmosphérique, sauf indication contraire. Dans les exemples, les quantités des ingrédients des compositions sont données en % pondéral par rapport au poids total de la composition.

### EXEMPLES :

### Exemple 1 : Formulations de compositions cosmétiques

Deux compositions cosmétiques ont été préparées de manière à ce qu'elles soient comparables quant à l'effet de l'ajout dans la composition du caprylate de glycérol, du phytate de sodium et du 1,3-propanediol.

Les compositions testées sont les suivantes :

**Tableau 1**

| INCl UE | Composition 1C comparative (% en poids) | Composition 1 selon l'invention (% en poids) |
|---|---|---|
| BENZYL ALCOHOL | 0,5 | 0,5 |
| CETEARYL ALCOHOL | 0,5 | 0,5 |
| DICAPRYLYL CARBONATE | 3 | 3 |
| C12-16 ALCOHOLS (and) PALMITIC ACID (and) HYDROGENATED LECITHIN (BIOPHILIC H de chez LUCAS MEYER COSMETICS) | 4 | 4 |
| HELIANTHUS ANNUUS SEED OIL | 3 | 3 |
| BUTYROSPERMUM PARKII BUTTER | 5 | 5 |
| SODIUM PHYTATE (dermofeel® PA-3 de la société DR STRAETMANS GmbH) | - | 0,1 |
| SCLEROTIUM GUM | 0,3 | 0,3 |
| XANTHAN GUM | 0,1 | 0,1 |
| GLYCERIN | 5 | 5 |
| PROPANEDIOL (Zemea® Propanediol de la société DUPONT TATE AND LYLE BIO PRODUCTS) | - | 3 |
| ETHYLIC ALCOHOL. | - | 3 |
| AQUA | QSP 100 | QSP 100 |
| GLYCERYL CAPRYLATE (dermosoft^{®} GMCY de la société DR STRAETMANS GmbH) | - | 0,5 |
| GLYCERYL STEARATE CITRATE | 1 | 1 |
| TOCOPHEROL | 0,025 | 0,025 |

| | | |
|---|---|---|
| QSP 100 : Quantité suffisante pour compléter à 100% du poids totale de la composition | | |

Les exemples suivants résument les résultats obtenus par application de ces compositions (comparative ou selon l'invention) sur la peau du visage, notamment au niveau du contour des yeux, d'un panel de six femmes:
0,30mL de chaque formule ont été appliqués pour chaque demi-visage, de façon randomisée L'évaluation a été réalisée immédiatement (2 minutes) après application versus peau nue, par des esthéticiennes expertes.

### Selon le protocole

Le panel a évalué d'une part la composition comparative *versus* peau nue, d'autre part, la composition selon l'invention *versus* peau nue. Comme indiqué ci-dessus, chaque application est réalisée sur demi-visage, l'autre demi-visage représentant ainsi la peau nue.

Dans l'intervalle entre les 2 applications, le panel s'est démaquillé parfaitement et s'est nettoyé le visage.

La notation des effets se fait sur une échelle en 7 points pouvant être positive et/ou négative, avec une mention « volontaire non concerné par le paramètre évalué ».

On recueille les données résumées dans les tableaux 2 à 5.

**Tableau 2 - Effet sur les stries de déshydratation**

| Paramètres évalués | | Formules | Cas concernés / Cas totaux | Graduation des effets | | | | | | | | | | | | | Note Globale | |
|---|---|---|---|---|---|---|---|---|---|---|---|---|---|---|---|---|---|---|
| | | | | Accentue la visibilité | | | | | | | Attenue la visibilité | | | | | | | |
| | | | | -6 | -5 | -4 | -3 | -2 | -1 | 0 | 1 | 2 | 3 | 4 | 5 | 6 | - | + |
| Stries de deshydratation | Yeux | Composition 1 selon l'invention | 6/6 | | | | | | | | | 1 | 4 | 1 | | | | 18 |
| | | Composition 1C comparative | | | | | | | | 1 | 2 | 3 | | | | | | 8 |
| | Visage | Composition 1 selon l'invention | 6/6 | | | | | | | | | | 3 | 3 | | | | 21 |
| | | Composition 1C comparative | | | | | | | | | 2 | 2 | 2 | | | | | 12 |

Il a été observé que la composition selon l'invention est plus performante (nettement supérieure) au niveau de l'atténuation des stries de déshydratation, notamment du visage et des yeux, que la composition comparative.

**Tableau 3 - Effet sur les rides et ridules**

| Paramètres évalués | | Formules | cas concernés /cas totaux | Graduation des effets | | | | | | | | | | | | | NOTE GLOBALE | |
|---|---|---|---|---|---|---|---|---|---|---|---|---|---|---|---|---|---|---|
| | | | | ACCENTUE la visibilité | | | | | | 0 | ATTENUE la visibilité | | | | | | | |
| | | | | -6 | -5 | -4 | -3 | -2 | -1 | | 1 | 2 | 3 | 4 | 5 | 6 | - | + |
| Rides et ridules | Paupière Inf. | Composition 1 selon l'invention | 6/6 | | | | | | | | | 2 | 3 | | 1 | | | 18 |
| | | Composition 1C comparative | | | | | | | | 4 | | 2 | | | | | | 4 |
| | Joues | Composition 1 selon l'invention | 4/6 | | | | | | | | | 3 | 1 | | | | | [9] |
| | | Composition 1C comparative | | | | | | | 1 | 3 | | | | | | | [-1] | |
| | Front | Composition 1 selon l'invention | 6/6 | | | | | | | 5 | | 1 | | | | | | 2 |
| | | Composition 1C comparative | | | | | | | | 6 | | | | | | | 0 | 0 |

La composition selon l'invention révèle une efficacité d'atténuation des rides et ridules nettement supérieure à la composition comparative sur les paupières inférieures, les joues et le front.

**- Tableau 4 - Effet sur les poches**

| Paramètres évalués | | Formules | cas concerné s/cas totaux | Graduation des effets | | | | | | | | | | | | | Note globale | |
|---|---|---|---|---|---|---|---|---|---|---|---|---|---|---|---|---|---|---|
| | | | | ACCENTUE la visibilité | | | | | | 0 | ATTENUE la visibilité | | | | | | | |
| | | | | -6 | -5 | -4 | -3 | -2 | -1 | | 1 | 2 | 3 | 4 | 5 | 6 | - | + |
| Relief cutané | Poches | Composition 1 selon l'invention | 6/6 | | | | | | 1 | 2 | 2 | | 1 | | | | -1 | 5 |
| | | Composition 1C comparative | | | | | | | 1 | 5 | | | | | | | -1 | |

La composition de l'invention permet d'atténuer le relief cutané, en particulier les poches, mieux que la composition comparative. Cette action peut s'expliquer par l'action de la composition sur les rides. Les effets notés avec la Composition 1 C sont quant à eux quasi nuls.

**Tableau 5 - Effet « tôle ondulée »'**

| Rendu « tôle ondulée » | Graduation des effets | | | | | | | Note Globale |
|---|---|---|---|---|---|---|---|---|
| | NON | Très léger | Léger | Moyen | Assez important | Important | Très important | |
| | 0 | 1 | 2 | 3 | 4 | 5 | 6 | |
| Composition 1 selon l'invention | 6 | | | | | | | 0 |
| Composition 1C comparative | 2 | | 2 | 1 | | 1 | | 12 |

La composition selon l'invention montre donc une efficacité importante sur les stries de déshydratation sans effet « tôle ondulée » contrairement à une composition comparative sans l'association de l'invention (caprylate de glycérol, phytate de sodium et 1,3-propanediol).

### Evaluation des aspects de formulations et d'application cosmétiques

Les compositions comparative et selon l'invention présentent des textures de crèmes gélifiées de densités assez importantes.

La composition comparative s'étale moins bien que la composition selon l'invention.

La composition de l'invention s'étale selon un film légèrement gras, soyeux et cireux sur le visage comme entre les doigts. Son étalement est donc tout à fait satisfaisant et agréable.

Contrairement à la composition comparative, la composition selon l'invention présente un meilleur toucher de peau après application. La composition selon l'invention laisse subsister un très léger film gras et soyeux.

## Revendications

1. Composition, notamment cosmétique, comprenant, dans un milieu physiologiquement acceptable, au moins du caprylate de glycéryl, du 1,3-propanediol et de l'acide phytique ou l'un de ses sels, ladite composition étant exempte de perlite.

2. Composition selon la revendication 1, **caractérisée en ce que** le sel d'acide phytique est un sel alcalin d'acide phytique.

3. Composition selon la revendication 2, **caractérisée en ce que** le sel alcalin d'acide phytique est le phytate de sodium.

4. Composition selon l'une quelconque des revendications précédentes, **caractérisée en ce qu'**elle comprend de 0,01% à 5 % en poids de caprylate de glycéryl par rapport au poids total de la composition.

5. Composition selon l'une quelconque des revendications précédentes, **caractérisée en ce qu'**elle comprend de 0,1% à 25 % en poids de 1,3-propanediol par rapport au poids total de la composition.

6. Composition selon l'une quelconque des revendications précédentes, **caractérisée en ce qu'**elle comprend de 0,01% à 1,5% en poids d'acide phytique ou l'un de ses sels, par rapport au poids total de la composition.

7. Composition selon l'une quelconque des revendications précédentes, **caractérisée en ce qu'**elle comprend en outre un alcool autre que le 1,3-propanediol, et de préférence comprend de l'alcool éthylique.

8. Composition selon l'une quelconque des revendications précédentes, **caractérisée en ce qu'**elle comprend de 0,1% à 25%, et de préférence de 0,1% à 10%, et encore de préférence de 0,1% % à 5 %, en poids d'alcool autre que le 1,3-propanediol, et de préférence d'alcool éthylique, par rapport au poids total de la composition.

9. Utilisation d'une composition, notamment cosmétique, comprenant, dans un milieu physiologiquement acceptable, au moins du caprylate de glycéryl, du 1,3-propanediol et de l'acide phytique ou l'un de ses sels, pour hydrater la peau.

10. Utilisation selon la revendication 9, **caractérisée en ce que** le sel d'acide phytique est le phytate de sodium.

11. Utilisation selon l'une quelconque des revendications 9 ou 10, **caractérisée en ce que** la composition est telle que définie dans l'une quelconque des revendications 1 à 8.

12. Méthode de soin cosmétique pour l'hydratation de la peau, **caractérisée en ce qu'**elle comprend l'application sur la peau d'une composition, notamment cosmétique, comprenant dans un milieu physiologiquement acceptable, au moins du caprylate de glycéryl, du 1,3-propanediol et de l'acide phytique ou l'un de ses sels.

13. Méthode selon la revendication 12, **caractérisée en ce que** le sel d'acide phytique est le phytate de sodium.

14. Méthode selon l'une quelconque des revendications 12 ou 13, **caractérisée en ce que** la composition est telle que définie dans l'une quelconque des revendications 1 à 8.

15. Méthode selon l'une quelconque des revendications 12 à 14, **caractérisée en ce que** la composition est appliquée sur une peau sèche et/ou âgée.

16. Méthode selon l'une quelconque des revendications 12 à 15, **caractérisée en ce qu'**elle comprend l'application de la composition sur une zone cutanée choisie parmi
- les mains,
- le visage en particulier le front, les joues, ou le contour d'un oeil (péri-oculaire), et en particulier la patte d'oie, la zone en dessous de l'oeil (poche), ou les paupières,
- le cou,
- les pieds,
- les jambes,
- les bras et avant-bras.

17. Méthode selon la revendication 16, **caractérisée en ce que** ladite zone cutanée présente de stries de déshydratation.

## Patentansprüche

1. Zusammensetzung, insbesondere kosmetische Zusammensetzung, umfassend, in einem physiologisch akzeptablen Medium, mindestens Glycerylcaprylat, 1,3-Propandiol, und Phytinsäure oder eines ihrer Salze, wobei die Zusammensetzung frei von Perlit ist.

2. Zusammensetzung gemäß Anspruch 1, **gekennzeichnet dadurch, dass** das Phytinsäure-Salz ein alkalisches Phytinsäure-Salz ist.

3. Zusammensetzung gemäß Anspruch 2, **gekennzeichnet dadurch, dass** das alkalische Phytinsäure-Salz Natriumphytat ist.

4. Zusammensetzung gemäß einem der vorhergehenden Ansprüche, **gekennzeichnet dadurch, dass** sie, im Verhältnis zum Gesamtgewicht der Zusammensetzung, von 0,1 bis 5 Gew.-% Glycerylcaprylat umfasst.

5. Zusammensetzung gemäß einem der vorhergehenden Ansprüche, **gekennzeichnet dadurch, dass** sie, im Verhältnis zum Gesamtgewicht der Zusammensetzung, von 0,1 bis 25 Gew.-% 1,3-Propandiol umfasst.

6. Zusammensetzung gemäß einem der vorhergehenden Ansprüche, **gekennzeichnet dadurch, dass** sie, im Verhältnis zum Gesamtgewicht der Zusammensetzung, von 0,01 bis 1,5 Gew.-% Phytinsäure oder eines ihrer Salze umfasst.

7. Zusammensetzung gemäß einem der vorhergehenden Ansprüche, **gekennzeichnet dadurch, dass** sie ferner einen Alkohol außer dem 1,3-Propandiol umfasst, und vorzugsweise Ethanol umfasst.

8. Zusammensetzung gemäß einem der vorhergehenden Ansprüche, **gekennzeichnet dadurch, dass** sie, im Verhältnis zum Gesamtgewicht der Zusammensetzung, von 0,1 bis 25, und vorzugsweise von 0,1 bis 10, und stärker bevorzugt von 0,1 bis 5 Gew.-% Alkohol außer dem 1,3-Propandiol umfasst, und vorzugsweise Ethanol.

9. Verwendung einer Zusammensetzung, insbesondere einer kosmetischen Zusammensetzung, die in einem physiologisch akzeptablen Medium mindestens Glycerylcaprylat, 1,3-Propandiol und Phytinsäure oder eines ihrer Salze umfasst, um die Haut zu hydratisieren.

10. Verwendung gemäß Anspruch 9, **gekennzeichnet dadurch, dass** das Phytinsäure-Salz Natriumphytat ist.

11. Verwendung gemäß einem der Ansprüche 9 oder 10, **gekennzeichnet dadurch, dass** die Zusammensetzung so wie in einem der Ansprüche 1 bis 8 definiert ist.

12. Verfahren der kosmetischen Pflege für die Hydratation der Haut, **gekennzeichnet dadurch dass** es das Auftragen einer Zusammensetzung, insbesondere einer kosmetischen Zusammensetzung, die in einem physiologisch akzeptablen Medium mindestens Glycerylcaprylat, 1,3-Propandiol und Phytinsäure oder eines ihrer Salze umfasst, auf die Haut umfasst.

13. Verfahren gemäß Anspruch 12, **gekennzeichnet dadurch, dass** das Phytinsäure-Salz Natriumphytat ist.

14. Verfahren gemäß einem der Ansprüche 12 oder 13, **dadurch gekennzeichnet, dass** die Zusammensetzung so wie in einem der Ansprüche 1 bis 8 definiert ist.

15. Verfahren gemäß einem der Ansprüche 12 bis 14, **dadurch gekennzeichnet, dass** die Zusammensetzung auf trockene und/oder gealterte Haut aufgetragen wird.

16. Verfahren gemäß einem der Ansprüche 12 bis 15, **dadurch gekennzeichnet, dass** es die Anwendung der Zusammensetzung auf einen Hautbereich umfasst, der ausgewählt wird aus:
- den Händen,
- dem Gesicht, insbesondere der Stirn, den Wangen, oder den Augenumrissen (periokular), und insbesondere Lachfältchen, dem Bereich unter den Augen (Tränensäcke), oder den Augenlidern,
- dem Hals,
- den Füßen,
- den Beinen,
- den Oberarmen und Unterarmen.

17. Verfahren gemäß Anspruch 16, **gekennzeichnet dadurch, dass** die betreffende Hautzone Dehydratationsfalten aufweist.

## Claims

1. Composition, in particular a cosmetic composition, comprising, in a physiologically acceptable medium, at least glyceryl caprylate, 1,3-propanediol and phytic acid or any of the salts thereof, said composition being free from perlite.

2. Composition according to claim 1, **characterized in that** the phytic acid salt is an alkaline phytic acid salt.

3. Composition according to claim 2, **characterized in that** the alkaline phytic acid salt is sodium phytate.

4. Composition according to any of the above claims, **characterized in that** it comprises 0.01% to 5% by weight of glyceryl caprylate with respect to the total weight of the composition.

5. Composition according to any of the above claims, **characterized in that** it comprises 0.1 % to 25% by weight of 1,3-propanediol with respect to the total weight of the composition.

6. Composition according to any of the above claims, **characterized in that** it comprises 0.01% to 1.5% by weight of phytic acid or any of the salts thereof with respect to the total weight of the composition.

7. Composition according to any of the above claims, **characterized in that** it further comprises an alcohol other than 1,3-propanediol, and preferably comprises ethyl alcohol.

8. Composition according to any of the above claims, **characterized in that** it comprises 0.1 % to 25%, and preferably 0.1 % to 10%, and more preferably 0.1 % to 5%, by weight of alcohol other than 1,3-propanediol, and preferably of ethyl alcohol, with respect to the total weight of the composition.

9. Use of a composition, in particular a cosmetic composition, comprising, in a physiologically acceptable medium, at least glyceryl caprylate, 1,3-propanediol and phytic acid or any of the salts thereof, and in particular sodium phytate, for moisturizing skin.

10. Use according to claim 9, **characterized in that** the phytic acid salt is sodium phytate.

11. Use according to any of claims 9 or 10, **characterized in that** the composition is as defined in any of claims 1 to 8.

12. Cosmetic care method for skin moisturization, **characterized in that** it comprises the application to the skin of a composition, in particular a cosmetic composition, comprising in a physiologically acceptable medium, at least glyceryl caprylate, 1,3-propanediol and phytic acid or any of the salts thereof.

13. Method according to claim 12, **characterized in that** the phytic acid salt is sodium phytate.

14. Method according to any of claims 12 or 13, **characterized in that** the composition is as defined in any of claims 1 to 8.

15. Method according to any of claims 12 to 14, **characterized in that** the composition is applied to dry and/or aged skin.

16. Method according to any of claims 12 to 15, **characterized in that** it comprises the application of the composition to a skin area chosen from
- the hands,
- the face, in particular the forehead, cheeks, or contour of an eye (periocular region), and in particular crow's feet, the area below the eye (bag), or the eyelids,
- the neck,
- the feet,
- the legs,
- the arms and forearms.

17. Method according to claim 16, **characterized in that** said skin area has dehydration lines.
